# EUROPEAN PATENT APPLICATION

(11) **EP 4 434 475 A1**
(43) Date of publication of application: **25.09.2024**
(21) Application number: 24151736.6
(22) Date of filing: 12.01.2024
(51) Int. Cl.: A61B 17/00

(54) **MEDICAL DEVICE CONTROL HANDLE WITH INTEGRATED FLUSH PORT/SEAL FEATURES**

(30) Priority: 13.01.2023 US 202363439057 P
(71) Applicant: Cook Medical Technologies LLC, Bloomington, IN 47402 (US)
(72) Inventor: HANSEN, Palle Munk, 4632 Bjaeverskov (DK)
(74) Representative: Leach, Sean Adam

(57) **Abstract**

Described are systems for hand-held control of medical device structures. The systems can include a control handle including a handle housing and an actuator movably mounted to the handle and attached to a control shaft. A sheath is attached to a distal end of the handle housing and has a lumen fluidly communicating with a distal opening of the sheath. The control shaft extends from the handle housing and into the lumen of the sheath. A medical device structure is attached to the control shaft and the actuator is operable by the hand of the user to cause movement of the control shaft distally relative to the handle housing, for example so as to deploy the medical device structure from the distal opening of the sheath, and proximally relative to the handle housing, for example so as to retract the medical device structure into the distal opening of the sheath. A seal element is mounted in the handle housing and the control shaft extends through the seal element. The seal element is cooperable with the control shaft to maintain a seal against the control shaft during the movement of the control shaft. A flush port is provided which fluidly communicates with the lumen of the sheath and can also fluidly communicate with a surface of the seal element. Handles useful in such systems, as well as related methods of manufacture and use, are also described.

## Description

### FIELD

Aspects of the present disclosure relate generally to medical devices. More particularly, certain aspects of the present disclosure relate to medical device control systems that have control handles with an actuator to manipulate a control shaft and attached medical device structure, and that incorporate a seal element associated with the control shaft and/or a flushing port for delivering a flushing medium into a lumen of a sheath associated with the control handles.

### BACKGROUND

The statements in this section merely provide background information related to the present disclosure and may not constitute prior art.

Medical device control systems often incorporate a handle for effective operation of components of the systems. A variety of operations are commonly desired during preparation and operation of such medical device systems, which can tend to make system structures, and their manufacture and use, somewhat complex. There remain needs for improved and alternative medical device control handles and associated medical device control systems, and methods for their manufacture and use. Desirably, the subject handles and systems will be effective and facile in use as well as practicable in manufacture. In at least some of its aspects, the present disclosure is addressed to such needs.

### SUMMARY

In one aspect, the present disclosure relates to a system for hand-held control of a medical device structure. The system includes a control handle including a handle housing and an actuator movably mounted to the handle housing and arranged for manual operation by a hand of a user. The system also includes a control shaft connected to the actuator and a sheath attached to a distal end of the handle housing. The sheath has a lumen extending to a distal opening of the sheath, and the control shaft extends from the handle housing and into the lumen of the sheath. A medical device structure is attached to the control shaft, and the actuator is operable by the hand of the user to cause movement of the control shaft distally relative to the handle housing and proximally relative to the handle housing. The movement of the control shaft distally relative to the handle housing can deploy the medical device structure from the distal opening of the sheath and the movement of the control shaft proximally relative to the handle housing can retract the medical device structure into the distal opening of the sheath. A seal element is mounted in the handle housing, with the control shaft extending through the seal element. The seal element is cooperable with the control shaft to maintain a seal against the control shaft during the movement of the control shaft. The system also includes a flush port opening in fluid communication with the lumen of the sheath. The flush port opening can be on the control handle. In some forms, the seal element can interrupt a fluid passage extending from the distal opening of the sheath, through the lumen of the sheath and into an interior space of the handle housing. The seal element, for example in the form of one or multiple seal discs, can include at least a first opening therein, with the control shaft received through the first opening and being longitudinally slidable and rotatable relative to the first opening.

In another aspect, the present disclosure relates to a method for preparing a system prior to use on a patient. The method includes providing a system as described above and/or elsewhere in the present disclosure, and passing a flush liquid into the flush port opening, through the lumen of the sheath, and out the distal opening of the sheath.

In still another aspect, the present disclosure relates to a method for treating a patient using a medical device. The method includes providing a system as described above and/or elsewhere in the present disclosure, and passing a flush liquid into the flush port, through the lumen of the sheath, and out the distal opening of the sheath. The method further includes, after the above-described step of passing a flush liquid, introducing the sheath into a vascular vessel of the patient, operating the actuator by hand to cause movement of the control shaft distally relative to the handle housing so as to deploy the medical device from the distal opening of the sheath, and treating the patient with the medical device.

In still another aspect, the present disclosure relates to a medical device control handle for hand-held control of a medical device structure that is connected to a control shaft and deployable from a lumen of a sheath. The medical device control handle includes a handle housing and an actuator movable relative to the handle housing and arranged for manual operation by a hand of a user, the actuator being connectable to the control shaft. The handle also includes at least one of, or both of: a) a seal assembly mounted in the handle housing and configured to maintain a seal against an outer surface of a control shaft for a medical device, the seal assembly including a seal body defining a lumen therein and an elastomeric seal element received against the seal body; and b) a flush port assembly mounted in the handle housing, the flush port assembly including a first flush port body portion defining a first flush lumen portion therein configured to fluidly communicate with a sheath lumen, a second flush port body portion extending transverse to the first flush port body portion and providing a flush port stem defining a second flush lumen portion therein, the second flush lumen portion intersecting and fluidly communicating with the first flush lumen portion. The seal assembly when included can also include a seal cap attached to the seal body so as to press the elastomeric seal element against the seal body portion. Handles that include both the seal assembly and the flush port assembly are preferred, and in such embodiments the flush port assembly and the seal assembly can be subcomponents of a flush port/seal assembly, beneficially where the seal body, the first flush port body portion, and the second flush port body portion are segments of an integral flush port/seal body. The handle housing can have one or more inwardly-directed walls that contact and positionally fix the flush port assembly or the seal assembly relative to the handle housing. The handle housing can be composed of at least, and in some forms only, a first handle housing portion and a second handle housing portion connected together.

Still further aspects of the present disclosure, as well as features and advantages thereof, will be apparent to those skilled in the art from the descriptions herein.

### BRIEF DESCRIPTION OF THE FIGURES

In order that the disclosure may be well understood, there will now be described various forms thereof, given by way of example, reference being made to the accompanying drawings. The components in each of the drawings may not necessarily be drawn to scale, rather emphasis is placed upon illustrating the principles of the invention. Moreover, like referenced numerals in different drawings designate corresponding or similar components or elements.
**FIG. 1** provides a partial cutaway, perspective view of one embodiment of a medical device control system in accordance with the present disclosure.
**FIG. 2** provides a partial cutaway top view of the medical device control system depicted in **FIG. 1****.**
**FIG. 3** provides a partial cutaway view of a distal region of a medical device control handle of the system depicted in **FIG. 1****,** showing internal features of the handle.
**FIG. 4** provides an exploded view of components of the distal region of the medical device control handle depicted in **FIG.** 3.
**FIG. 5** provides a partial cutaway view showing of components of the system depicted in **FIG. 1** in a partially disassembled state.
**FIG. 6** provides a partial cutaway view of components of another embodiment of a medical device control system in accordance with the present disclosure.

### DETAILED DESCRIPTION

Reference will now be made to certain embodiments, some of which are illustrated in the drawings, and specific language will be used to describe the same. It will nevertheless be understood that no limitation of the scope of the disclosure herein is thereby intended. Any alterations and further modifications in the described embodiments and any further applications of the principles as described herein are contemplated as would normally occur to a person skilled in the art to which the embodiments relate.

As disclosed above, certain embodiments of the present disclosure relate to systems for hand-held control of a medical device structure, to handles and other components thereof, and to methods of their manufacture and use.

For the purpose of the present disclosure, the term "proximal" refers to a direction that is generally towards a user (e.g., physician) during a medical procedure, while the term "distal" refers to a direction that is generally towards a target site within a patient's anatomy during the medical procedure.

For the purpose of the present disclosure, the terms "at least one" and "one or more of" an element are used interchangeably and may have the same meaning. These terms, which refer to the inclusion of a single element or a plurality of the elements, may also be represented by the suffix "(s)" at the end of the element. For example, "at least one metal", "one or more metals", and "metal(s)" may be used interchangeably and are intended to have the same meaning.

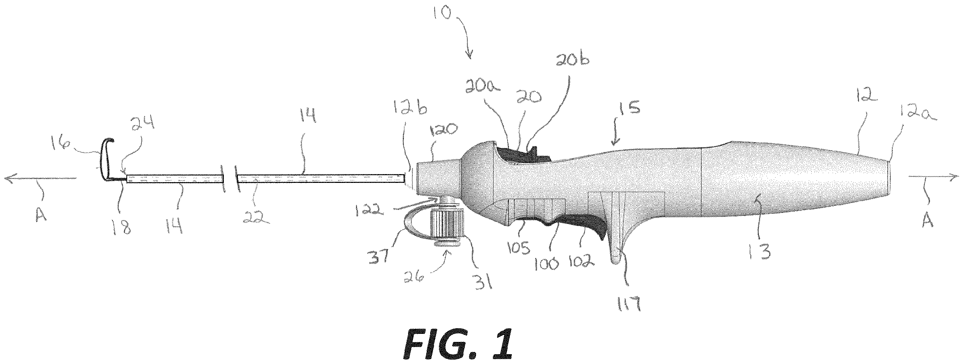

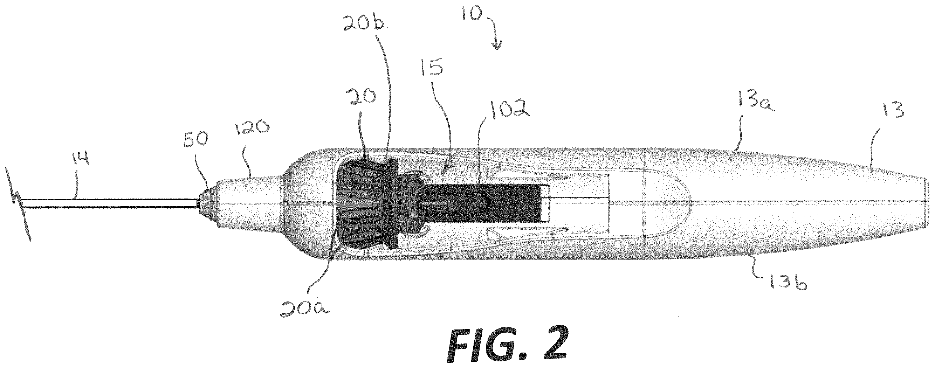

With reference now to **FIGs. 1** and **2**, shown are a partial cutaway, perspective view and a partial cutaway top view, respectively, of one embodiment of a medical device control system **10** in accordance with the present disclosure. System **10** generally includes a medical device control handle **12** having a proximal end **12a** and distal end **12b** to which is attached a sheath **14** (which can, for instance, be a catheter). System **10** includes a snare element, such as a vascular snare element, having a snare loop **16,** although in other forms the snare element may include hooks or multiple loops, or the snare element may instead be another medical device structure, for example all of or a portion of another gripping member such as a multi-pronged clamp or clasp. The snare loop **16** or other medical device structure can be connected to a longitudinally extending control shaft **18,** which in turn can be connected to an actuator such as an actuator button **20.** Snare loop **16** and control shaft **18** can each be made of a single component or multiple components, can be made of any suitable material such as a polymeric and/or metal material, and can be or include solid cross-sectional (e.g. solid wire structures) or hollow parts (e.g. cannula structures), as known in the art. As well, snare loop **16** and control shaft **18** may be a single integral component. In some configurations, snare loop **16** is a braided wire loop.

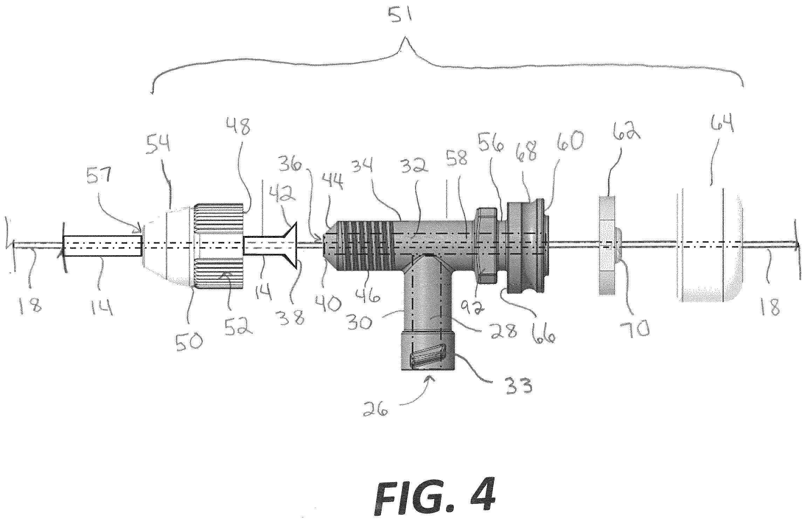

In some forms, the longitudinally extending control shaft **18** extends to directly connect with actuator button **20**, for example being received and secured within actuator button, for example by a locking mechanism, an adhesive, or both, or other techniques. The medical device control handle **12** includes a handle housing **13** that defines a cutout **15** that creates a movement space therein. The actuator button **20** is disposed within the cutout **15** and connected to the control shaft **18.** The actuator button **20** may be movable within the movement space defined by the cutout **15** along the longitudinal axis **(A-A)** of the system **10** and/or rotatable within the movement space defined by the cutout **15,** for example with a rotational axis also along the longitudinal axis **A-A.** The actuator button **20** can be movable, for example slidable, along the longitudinal axis **A-A** independent of rotation of the actuator button **20.** The longitudinal movement of the actuator button **20** can correspondingly longitudinally move the control shaft **18** and the snare loop **16** or other medical device structure attached to control shaft **18,** and/or the rotation of the actuator button **20** can correspondingly rotate the control shaft **18** and the snare loop **16** or other medical device structure attached to the control shaft **18.** The longitudinal movement and/or rotational movement associated with the actuator button **20** may be incorporated such that one finger of the user is capable of controlling both the longitudinal and/or rotational movement of the medical device by a sliding motion and/or a scrolling motion, respectively. In some forms, the actuator button **20** may be a thumb wheel capable of being manipulated solely by the thumb finger of the user (e.g., physician) in order to move the control shaft **18** and the snare loop **16** (or other medical device structure) both longitudinally and rotationally, for example through the lumen **22** of sheath **14** and/or within the patient when deployed out of the end opening **24** of sheath **14.** When desirable, the thumb wheel or other actuator button **20** as shown for example in **FIGs. 2** and **4** may include one or more fins **20a** and/or an indented edge **20b** configured to fit a thumb finger to provide for additional gripping during rotational and longitudinal movements. When the actuator button **20** is a thumb wheel the fins **20a** may be disposed around the circumference of the wheel, and/or the button **20** can define a generally cylindrical exterior profile.

The handle includes a handle housing **13** that may be a singular unified component or include a plurality of components, such as an upper body portion and a lower body portion, or such as a left-side body portion and a right-side body portion, that are attached together by any suitable mechanism, including for example by correspondingly-located pins and holes for receiving the pins, gluing, welding, melt bonding, combinations thereof, or the like. The dimensions of the handle housing **13** may be customized in order to fit a specific predetermined hand size or be optimized such that various hand sizes are capable of obtaining a firm grip when using the handle **12.** Additional ergonomic features may be included in the elongate handle housing as well without exceeding the scope of the present disclosure.

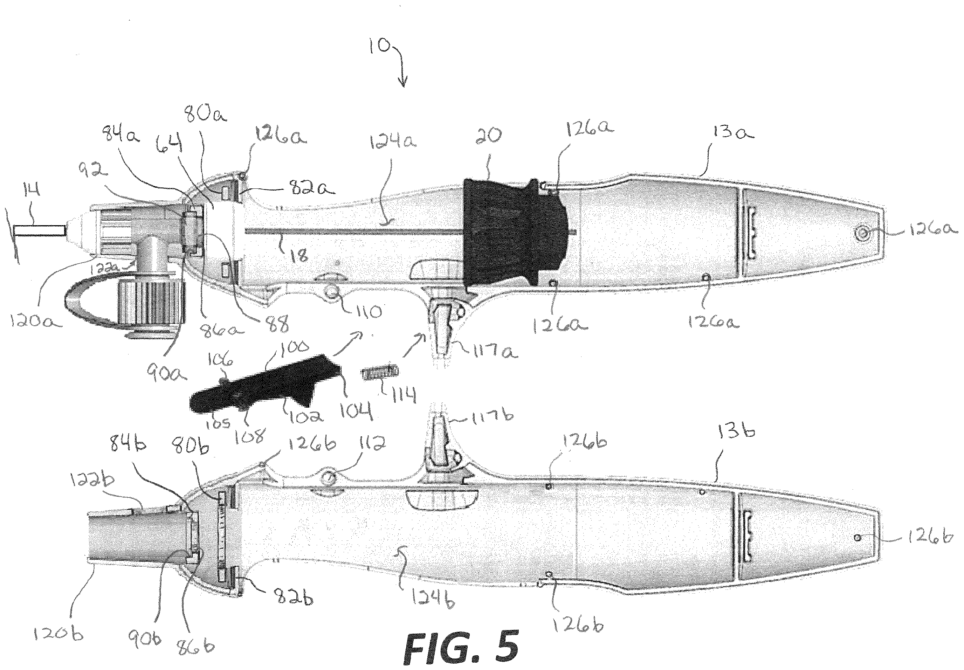

The control shaft **18** in the illustrated embodiment extends through the lumen **22** of sheath **14** and into the handle **12.** When, as illustrated, the actuator button **20** is in its forward (distally-positioned) position, the snare loop **16** is deployed from the distal opening **24** of the sheath **14.** When the actuator button **20** is in its rearward (proximally-positioned) position (see e.g. **FIG. 5**), the snare loop **16** is positioned within the lumen **22** of sheath **14.** Thus, button **20** can be translated distally and proximally to deploy and retract the snare loop **16,** respectively, from and into the lumen **22** of sheath **14.** The actuator button **20** can also be rotated to rotate the snare loop **16,** for example after the snare loop **16** is deployed distally out of the distal opening **24** of sheath **14.**

In some forms, as illustrated, the snare loop **16** extends at an angle relative to the longitudinal axis of the control shaft **18** when deployed distally out of the distal opening **24** of sheath **14,** and the actuator button **20** can be rotated to rotate the angled snare loop **16** to different radially positioned capture orientations. In use, this can facilitate positioning the snare loop **16** around a portion of an implanted medical device (which can be a vascular filter, such as a vena cava filter) to be retrieved, after which the actuator button **20** can be moved proximally to draw the snare loop **16** partially into the lumen **22** of sheath **14** so as to tighten the snare loop **16** around the implanted medical device. In some forms, the implanted medical device can be drawn into the lumen **22** of sheath **14** for removal from the patient. In other forms, the system **10** may be used in conjunction with another sheath or catheter, and the system **10** may be used to draw the implanted medical device into the other sheath or catheter. These and other modes of use for system **10** may be employed.

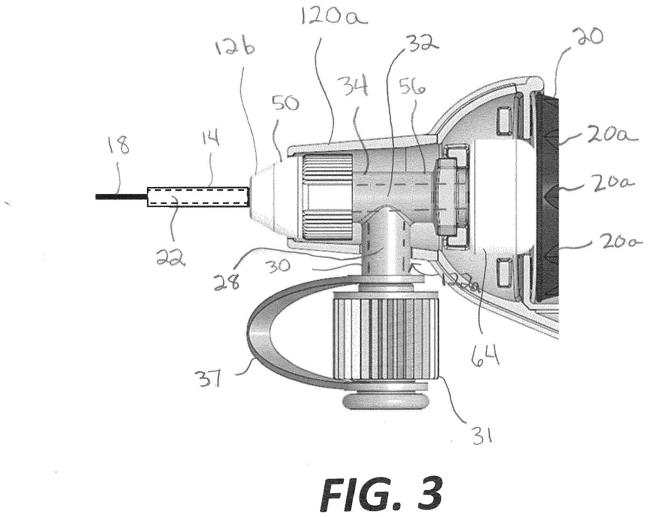

With continued reference to **FIGs. 1** and **2** now also in connection with **FIGs. 3** and **4****,** aspects of the system **10** relating to a flush port and/or seal arrangement will now be described. The illustrated control handle **12** incorporates a flush port opening **26** on the handle **12** that fluidly communicates with the lumen **22** of sheath **14.** In the embodiment shown, prior to use of the system **10** on a patient, the flush port opening **26** can be utilized to introduce a flush liquid, such as water or saline, to pass into and distally through lumen **22** and exit distal opening **24** of sheath **14,** for example to flush air from the lumen **22** (and in particular to flush the generally annular space between the outer surface of the control shaft **18** and the inner surface of sheath **14** that defines the lumen **22**). The flush port opening **26** communicates with a flush port lumen **28** defined by a flush port stem **30,** with the flush port lumen **28** and stem **30** extending transversely (e.g. perpendicularly) to the longitudinal axis **A-A** of the control handle **12** and/or system **10.** The flush port lumen **28** intersects and fluidly communicates with a longitudinal flush lumen **32** defined by a longitudinally-extending flush body portion **34.** The longitudinal flush lumen **32** in turn fluidly communicates with the lumen **22** of sheath **14,** so as to direct flush liquid introduced through flush port opening **26** into the lumen **22** of sheath **14.** The longitudinal flush lumen **32** also fluidly communicates with a surface of a seal element **62,** to be discussed further below. In the illustrated embodiment, the longitudinally-extending flush body portion **34** defines a distal opening **36** of the longitudinal flush lumen **32** that opens into the sheath lumen **22.** This can be provided by a connection of the proximal end **38** of the sheath **14** to the distal end **40** of the longitudinally-extending flush body portion **34.** The embodiment shown includes an outwardly flared proximal end region **42** of the sheath **14** that mates with a distally tapered region **44** of the longitudinal flush body portion **34.** As well, the outer surface of the longitudinally-extending flush body portion **34** defines a threaded region **46** (see in particular **FIG. 4**) that threadably meshes with a threaded region **48** on the interior surface of the connector cap **50.** Connector cap **50** can thereby be threaded onto the longitudinally-extending flush body portion **34** using threaded regions **46** and **48** to threadably connect the connector cap **50** and flush body portion **34,** and in so doing the connector cap **50** can press the flared proximal end region **42** of sheath **14** against the distally tapered region **44** of flush body portion **34** to create a seal between them. The connector cap **50** can be sized and include inner surfaces contoured to achieve such a seal. The connector cap **50** may also include longitudinally-extending grip ridges **52** on its external surface to aid in threading operations during assembly and/or a distally-tapering distal region **54** that can define inner surfaces contoured to engage and press the flared proximal end region **42** to create the seal as discussed above when connector cap **50** is threaded onto flush body portion **34.** The connector cap **50** also defines a distal cap opening **57** through which sheath **14** extends. A flush port cap **31** can also be provided that is removably attachable to the flush port stem **30** to cover and/or seal the flush port opening **26,** and in such cases a strap **37** can also be provided that connects the flush port cap **31** to the flush port stem **30.** For these purposes external surfaces of the flush port stem **30** can define a threaded luer or other threaded structure **33** (see **FIG. 4**) and internal surfaces of the cap **31** can define a corresponding threaded structure (not shown) for threaded attachment of the cap **31** to the stem **30.** The flush port cap **31** can be removed from flush port stem **30** to enable a flushing operation as described herein and/or can be attached to the flush port stem **30** (e.g. re-attached after such removal and flushing) so as to seal flush port opening **26,** which during use of the system **10** to treat a patient can also prevent any bodily liquid (e.g. blood) that backflushes proximally through lumen **22** of sheath **14** and into the flush port lumen **28** from exiting the flush port opening **26** and/or prevent the entry of air into the flush port opening **26.**

The illustrative system **10** also includes a sealing arrangement to provide a seal against control shaft **18,** including during movement of control shaft **18** caused by movement of the actuator button **20.** Such seal can serve to prevent backflushing of patient bodily fluid (e.g. patient blood) proximally through the lumen **22** of sheath **14** and into an interior space of the medical device control handle **12,** for example an interior space from which the bodily fluid can leak out of an opening of the handle housing **13.** In the illustrated embodiment, this sealing arrangement is advantageously provided by structures integrally connected to the flush port opening **26** and its associated components as discussed above, although this is not necessary in other embodiments. Particularly, as illustrated, a proximal seal body portion **56** is connected to the flush body portion **34,** and can in a beneficial embodiment be formed as an integral, monolithic piece with the flush body portion **34** and the flush port stem **30.** The proximal seal body portion **56** defines a proximal seal body portion lumen **58** that fluidly communicates with the longitudinal flush lumen **32** and that can be axially aligned therewith.

Proximal seal body portion **56** defines a proximal end surface **60** against which is received a seal element **62,** for example in the form of a seal disc as illustrated. The seal element can sealingly interrupt a fluid passage extending from the distal opening **24** of the sheath **14,** through the lumen **22** of the sheath **14** and into an interior space of the handle housing 13, for example such interior space being a movement space in which actuator **20** moves longitudinally and/or rotates as in the illustrated embodiment. Such fluid passage can in some forms also include a lumen defined by a flush body (e.g. flush body portion **34** and its lumen **32**) and/or a lumen defined by a seal body (e.g. proximal seal body portion **56** and its lumen **58**). The seal disc or other seal element **62** can be formed from a resilient material, for example an elastomeric material, that can conform to the outer surface of control shaft **18.** For example, the seal disc or other seal element **62** can be formed from a polymeric elastomer such as silicone or rubber. The seal disc or other seal element **62** can provide a seal barrier that extends across the seal body lumen **58.** The seal disc or other seal element **62** can be pressed (and potentially compressed) against the proximal end surface **60** by a seal cap **64** attached to the proximal seal body portion **56,** for example by press fitting. For these purposes, the proximal seal body portion **56** can define a shoulder **66** and/or groove **68** that can cooperate with interior surfaces of the seal cap **64** to secure the seal cap **64** to proximal seal body portion **56** when press fit thereon. In certain embodiments, a seal element **62** in the form of a seal disc can define an annular ring portion **70** that extends proximally from adjacent surfaces of the seal disc and that surrounds an inner portion of the seal disc through which the control shaft **18** passes in the assembled system **10,** with such inner portion forming a seal against the outer surface of the control shaft **18** as discussed herein. As shown for example in **FIG. 4****,** a portion of the control shaft **18** can be suspended in the handle housing **13** between the actuator **20** and the seal element **62,** and it will be understood that the portion of the control shaft **18** so suspended can shorten as actuator **20** is moved distally and lengthen as actuator **20** is moved proximally. In some embodiments, the seal element **62,** for example a sealing disc, will define a slit, multiple slits, a weakened portion such as a score, and/or multiple such weakened portions, to provide or facilitate the creation of an opening in the seal element **62** through which control shaft **18** extends in the system **10.** In some forms, a single seal disc or other seal element **62** can be provided through which control shaft **18** extends. In other forms, multiple discrete seal discs or other seal elements **62** can be provided through which control shaft **18** extends, for example with such seal discs or other seal elements being in a configuration stacked against one another. These and other seal arrangements will be apparent to those skilled in the field and may be used within handles and systems of the present disclosure.

The control shaft **18** can be frictionally translated proximally and distally through and against seal element **62** and/or can be frictionally rotated within and against seal element **62,** while maintaining a seal between the outer surface of control shaft **18** and the proximal seal body portion **56.** In this manner, any bodily fluid, for example blood, that passes into the lumen **22** of the sheath **14** from its distal opening **24** (e.g. in the annular space between the control shaft **18** and the surface defining the lumen **22** of the sheath **14**), is prevented from passing proximally past the seal element **62** and into an interior space of the medical device control handle **12.** Also, when a flush liquid is introduced into flush port opening **26** under pressure, the flush liquid may pass through flush port lumen **28** and into proximal seal body portion lumen **58** and against a surface of the seal element **62.** In such cases the seal element **62** can also prevent flush liquid from passing proximally past the seal element **62** and into interior spaces of the medical device control handle **12.** As well, the friction between the control shaft **18** and seal element **62** can facilitate a smoother and more tactile operation of the actuator button **20** as it is translated distally and proximally by a user and/or is rotated by a user.

While medical snare embodiments above have sometimes been described having a single snare loop **16,** it will be understood that in other embodiments other medical device structures, for example other snare capture elements that may include multiple snare loops or hooks, or all or part of another gripping member such as a clamp or clasp with multiple prongs (e.g. that may be urged toward one another by retracting a part of the gripping member into lumen **22** of sheath **14**) may be used in the place of the snare loop **16.** Such other medical device structures can be connected to the control shaft **18** and can be deployed from and retracted into the lumen **22** of the sheath **14** using movement of the actuator button **20** along the longitudinal axis **A-A** and/or can be rotated by rotating the actuator button **20,** as described above.

Referring now also to **FIG. 5** in conjunction with **FIGs. 1-4****,** shown in **FIG. 5** is a partial cutaway view of components of the system **10** in a partially disassembled condition to further illustrate aspects of the system **10.** In the embodiment shown, the medical device control handle **12** has a handle housing **13** formed from multiple handle housing portions, and in particular a first handle housing portion **13a** and a second handle housing portion **13b.** As will be discussed, the housing portions **13a** and **13b** are sized and have internal structures configured to fix the position of internal component(s) of the handle **12** relative to the handle housing **13** when attached together to form the handle housing **13.** In this regard, in respect of the flush port/seal assembly **51** (see e.g. **FIG. 4****)** including the connector cap **50,** the flush port stem **30,** the flush body portion **34,** the proximal seal body portion **56,** the seal element **62,** and the seal cap **64,** the handle housing portions **13a** and **13b** define respective inwardly-directed walls **80a** and **80b** that in the assembled handle **13** contact outer wall portions of seal cap **64** and hold seal cap **64** in a radially-secured position within the handle housing **13** spaced from outer walls of the housing **13** defined by the housing portions **13a** and **13b.** The walls **80a** and **80b** can in some aspects form a collar that surrounds outer wall portions of seal cap **64.** As well, the housing portions **13a** and **13b** define respective inwardly-directed walls **82a** and **82b** that define an opening in the assembled handle **13** that is smaller than the greatest external dimension of cap **64** and that is positioned proximally of such greatest external dimension, so as to restrict proximal movement of the seal cap **64** and thereby the flush port/seal assembly **51.** The inward edges of the walls **82a** and **82b** that define such opening can contact the cap **64** in the assembled handle housing **13** for these purposes. The handle housing portions **13a** and **13b** also define respective inwardly-directed walls **84a** and **84b** that in the assembled handle **13** have proximally-facing surface portions that contact distally-facing surface portions of the seal cap **64** and that secure seal cap **64** and thereby the flush port/seal assembly **51** against distal movement within the assembled handle **13.** As illustrated, the walls **84a** and **84b** can also define an opening sized to contact and cradle external surfaces of the proximal seal body portion **56** to radially secure the position of proximal seal body portion **56** within the assembled handle **13.** In some forms the walls **84a** and **84b** can form a collar in contact with and surrounding external surface of the proximal seal body portion **56.** In the particular illustrated embodiment, walls **84a** and **84b** define a first opening within inward wall edges **86a** and **86b** that contact and cradle (e.g. form a collar that surrounds) a first, smaller-dimension segment **88** of the proximal seal body portion **56** and a second opening within inward wall edges **90a** and **90b** that contact and cradle (e.g. form a collar that surrounds) a second, larger-dimension mount segment **92** of proximal seal body portion **56.** The larger-dimension mount segment **92** can in some forms have a non-circular external profile in cross-section, for example such external profile can be polygonal such as rectangular or square. In this manner, the engagement of inward wall edges **90a** and **90b** with surfaces of the non-circular external profile of the larger-dimension mount segment **92** can secure the flush port/seal assembly **51** against rotational movement relative to the handle housing **13.** As well, walls **84a** and **84b** as shown include a first wall segment that extends generally along the longitudinal axis **A-A** and a second wall segment that extends perpendicular thereto (e.g. forming an "L" shape in cross-section), and together provide distally-facing surfaces that contact proximally-facing surface portions of larger-dimension mount segment **92** to secure the flush port/seal assembly against proximal movement relative to the handle housing **13,** as well as proximally-facing surfaces that contact distally-facing surfaces of the shoulder **66** and/or of seal cap **64,** so as to secure the flush port/seal assembly **51** against distal movement relative to the handle housing **13.** In some forms, the flush port stem **30,** the flush body portion **34,** and the proximal seal body portion **56,** can be subcomponents of a unitary, integral flush port/seal body member as illustrated for example in **FIG. 4****.**

While certain embodiments and structures relating to the incorporation of internal walls of the handle housing **13** to secure the position of the flush port/seal assembly **51** have been discussed above, it will be understood that in broader aspects the present disclosure contemplates those discussed and/or other internal wall structures of the handle housing **13** that secure the assembly **51** in position relative to the handle housing **13,** for example including securing the assembly **51** against proximal and/or distal movement relative to the handle housing **13,** and/or against rotational movement relative to the handle housing **13,** and/or against lateral movement relative to the handle housing **13** (e.g. maintaining components thereof radially secured at a consistent spaced distance from inner surfaces of laterally-adjacent outer walls the handle housing **13).** This can be achieved for example by providing one or a plurality of inwardly-extending wall structure(s) of the handle housing **13** that contact external surfaces of the flush port/seal assembly **51** to secure the position thereof. Where the handle housing **13** includes multiple pieces (e.g. two, three or four pieces) that mate and are attached together to form the housing **13,** inwardly extending wall structure(s) on each piece can coordinate to contact the external surfaces of the assembly **51** to provide the positional securement of the assembly **51** (e.g. securement against rotational, proximal, distal, and/or lateral movement) relative to the handle housing **13.**

With particular reference to **FIG. 5** along with **FIGs. 1** and **2****,** the handle **12** also includes a locking member **100.** The locking member **100** is connected to the handle housing **13** and movable between a locked position and unlocked position. When in the locked position, the locking member **100** may be in contact with the actuator button **20** and be configured to restrict the movement of the actuator button **20** along the longitudinal axis **A-A.** For example, in the illustrated embodiment, when the actuator button **20** is in a distal position at which the snare loop **16** (or other medical device) is positioned within the lumen **22** of the sheath **14,** movement of the locking member **100** to the locked position can be achieved by pressing on trigger portion **102** (e.g. with an index finger) to pivot the locking member **100** in a first direction and drive the proximal end **104** into the interior of handle housing **13** to a position distal of and that interferes with distal movement of the actuator button **20.** In some forms, such a locked position can include contact between the proximal end **104** of the locking member **100** and the actuator button **20** (e.g. with a distal face portion of actuator button **20).** The handle housing **13** can, for example by contours of the housing portions **13a** and **13b,** define an opening in a location corresponding to all or a portion of the locking member **100,** to allow travel of a portion of the locking member including the proximal end **104** into and out of the interior of the handle housing **13** for these purposes. To move the locking member **100** to the unlocked position from the locked position, a user can press on an unlocking portion **105** (e.g. with their index finger) to pivot the member **100** in a second direction opposite the first direction so as to drive the proximal end **104** of member **100** in a direction outward of the interior of the handle housing **13** to eliminate interference with the actuator button **20** and allow the actuator button **20** to be moved distally within the movement space defined by cutout **15,** so as to deploy the snare loop **16** or other medical device structure out of the distal opening **24** of the lumen **22** of the sheath **14.**

In the illustrated embodiment, to secure the locking member **100** to the housing **13,** the locking member **100** defines laterally-extending posts **106** and **108** and the housing portions **13a** and **13b** respectively define corresponding openings **110** and **112** to receive the posts **106** and **108** when the portions **13a** and **13b** are attached together to form the housing **13.** The posts **106** and **108** can rotate within the openings **110** and **112** to allow the locking member **100** to pivot in the first and second directions and between the locked and unlocked positions, as discussed above. Also in the embodiment shown, a spring **114** is included in the handle **12** with a first end positioned against the proximal surface **116** of locking member extension **118** and a second end captured in a cavity formed in the handle housing **13.** For example, the handle housing may define a finger rest **117,** for example in the form of a fin or other projecting member extending transverse to the longitudinal axis **A-A,** and in some forms the cavity in which the second end of spring **114** is captured can be defined within the fin or other finger rest **117,** for instance by finger rest portions **117a** and **117b** of housing portions **13a** and **13b.** The spring **114** is positioned and configured to bias the locking member **100** in the locked position when forcibly moved from the unlocked position to the locked position and to bias the locking member **100** in the unlocked position when forcibly moved from the locked position to the unlocked position. The spring **114** can transition between alternately curved conditions when providing such biasing functions. The finger rest **117** in beneficial embodiments will occur on a side of the handle housing **13** that is opposite the side in which the cutout **15** is defined. In this manner, in a one-handed manual operation, a user can grip around the handle **12** and position a thumb of their hand on the actuator button **20** (e.g. a thumb wheel) with another finger (e.g. the index finger) of that hand positioned distal of and against the finger rest **117** and potentially still another finger (e.g. the middle finger) of that hand positioned proximal of and against finger rest **117,** facilitating a stable grip on the handle **12** during proximal and distal movement of the actuator button **20** with the thumb. Also, when locking member **100** is included, such a hand position at the same time facilitates use of the index finger positioned distal of the finger rest **117** to press on locking member **100** to move it between its locked and unlocked positions, e.g. as discussed above. Also, the finger rest **117** in some embodiments can occur on the same side of the handle housing **13** from which flush port stem **30** extends, and potentially being generally aligned with finger rest **117** about the circumference of the handle **12** considered in its longitudinal profile.

As noted above, the housing **13** of the illustrated embodiment has two housing portions, **13a** and **13b.** The illustrated portions are generally in the form of coordinating housing shells that come against one another along contours of their outer edges. The housing portions **13a** and **13b,** or other housing portions of multiple-piece housings herein, can be secured to one another using any suitable technique or mechanism, including for example by correspondingly-located holes and pins defined by the respective housing portions, by adhesives or bonding agents, by welding (e.g. ultrasonic welding), or combinations thereof, or other means. These and other attachment techniques will be understood as suitable by skilled persons given the disclosures herein. In addition or alternatively, the housing portions **13a** and **13b,** or other housing portions of multiple-piece housings herein, can in some forms each be a unitary, integral piece, for example a unitary, integrally molded (e.g. injection molded) piece.

In other features, the housing **13** has a tapered distal cone portion **120** defined by hemicone portions **120a** and **120b** of housing portions **13a** and **13b,** respectively. In some embodiments as shown, a proximal region of the connector cap **50** extends out of and distally beyond the distalmost end of the cone portion **120.** The handle housing **13,** and in the shown embodiment the cone portion **120** thereof, also defines an opening **122** for receiving the flush port stem **30** such that stem **30** resides partially within and partially exterior of the handle housing **13.** Opening **122** in the embodiment shown is provided by semi-circular cutouts **122a** and **122b** in housing portions **13a** and **13b,** respectively. Housing **13** also has interior surfaces that are complementary to the external profile of actuator button **20** so as to provide a longitudinally-extending slideway that guides the longitudinal movement of actuator button **20** relative to housing **13.** Such interior surfaces for the slideway can include for example concavely-curved lateral surfaces **124a** and **124b** of housing portions **13a** and **13b,** respectively, that are complementary to convexly-curved outer surfaces on the actuator button **20.** Housing portion **13a** defines a plurality of holes **126a** (for example defined by receptacle bosses) and housing portion **13b** defines a plurality of correspondingly-located pins **126b** that friction and/or snap fit within holes **126a** to attach housing portions **13a** and **13b** to one another. Such attachments can be sufficient for the finished handle **12** or system **10,** or other attachment techniques can be used instead of or in addition to the use of holes **126a** and pins **126b.**

The medical device control handles **12** and systems **10** incorporating them, including the various parts contained therein, may be formed from polymeric or plastic materials, ceramic materials, metals or metal alloys, and/or combinations thereof. The materials are selected so that they exhibit desirable or required performance characteristics, such as biocompatibility, flexibility, and strength to name a few. The polymeric or plastic materials may include one or more thermoplastic materials or thermoset materials, individually or in combination. Several examples of suitable polymeric or plastic materials may include but not limited to polyamides (e.g., nylons), polyimides, polyethylenes, polyurethanes, polyethers, polyesters, acrylonitrile butadiene styrene (ABS), and mixtures or copolymers thereof. In one form, the handle housing **13** of the handle **12** is formed from ABS. Any metal parts used within the system **10** may be formed from, without limitation, stainless steel, brass, a nickel-titanium allow such as nitinol, or a combination thereof.

In accordance with some aspects, the components described herein can be assembled in any suitable fashion or order to provide the system **10.** However, in certain preferred forms, a core assembly will first be assembled that includes the snare loop **16** or other medical device structure, the sheath **14,** the flush port/seal assembly **51,** the actuator button **20,** and the control shaft **18** attached to the snare loop **16** or other medical device and extending through the lumen **22** of the sheath **14** and through the flush port/seal assembly **51** and proximally attached to the actuator button **20.** This core assembly can then be associated with the handle housing **13,** for example by assembling multiple handle housing portions (e.g. housing portions **13a** and **13b** as discussed above) over and around the actuator button **20** and the flush port/seal assembly **51,** and a segment of the control shaft **18** extending between the actuator button **20** and the flush port/seal assembly **51,** and connecting such handle housing portions to one another e.g. as discussed herein. The assembly and connection of such multiple handle housing portions can beneficially secure the position of the flush port/seal assembly **51** relative to the handle housing **13** with inwardly-directed wall structures as discussed hereinabove. These assembly operations, and the system **10** component configurations that facilitate them, provide for a particularly convenient and practicable manufacture of the system **10.** Also, skilled persons will understand that where locking member **100** and spring **114** are included, these can be placed and captured between the multiple handle housing portions (e.g. **13a** and **13b)** as such portions are assembled together and connected to one another, to provide their positions and functions as described hereinabove.

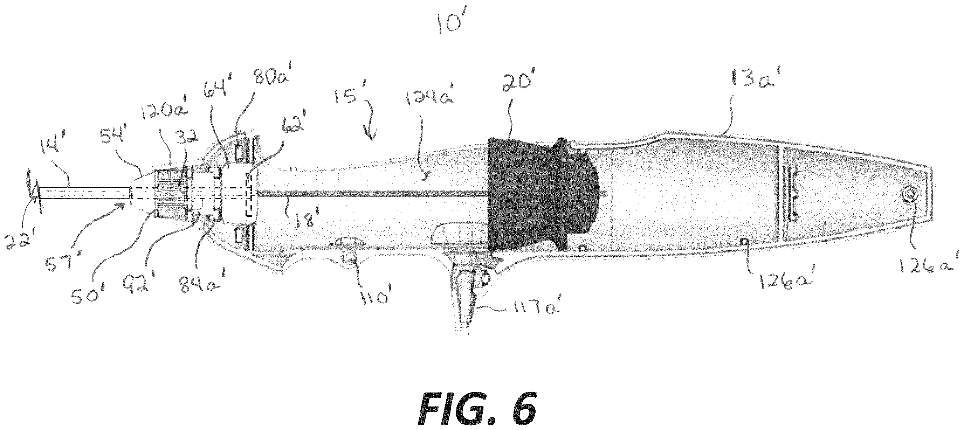

Referring now to **FIG. 6****,** shown are components of an alternate medical device control system **10'** that can have components similar to those in system **10** discussed above which are similarly numbered in **FIG. 6** along with a prime symbol " ' ", except system **10'** is equipped with a seal assembly but does not include a flush port assembly integral with the seal assembly. It will be understood that system **10'** will include a second handle housing portion (not shown) correspondingly shaped to and that is attached to handle housing portion **13a'** in forming the handle of system **10',** similar to housing portions **13a** and **13b** as discussed in conjunction with system **10** above. As shown in **FIG. 6****,** the seal assembly of system **10'** includes a seal body portion **56'** that defines an internal lumen **58',** a seal element **62'** such as a seal disc, and a seal cap **64'** attached, for example press fitted, to the seal body portion **56'** and pressing the seal element against the seal body portion **56',** potentially compressing the seal element against the seal body portion **56'.** The seal assembly of system **10'** is similarly positionally secured within the medical device control handle of system **10'** by internal wall structures of the handle housing, for example walls **80a'** and/or **84a'** and corresponding inwardly-directed walls on the included second handle housing portion, as discussed above in conjunction with system **10** and its corresponding inwardly-directed support wall structures. In system **10',** the housing portion **13a'** can define a further inwardly-directed wall **85'** positioned distal of enlarged seal body portion **92'.** Wall **85'** (along with a corresponding inwardly-directed wall on the included second handle housing portion) can abut a distal face of the enlarged seal body mount portion **92'** to prevent distal movement of the seal assembly and/or can contact and cradle (e.g. to form a collar that surrounds) correspondingly-located external surfaces of the seal body portion **56'** to hold the seal assembly in a radially-secured position within the handle housing spaced from outer walls of the housing.

When system **10** or **10'** is equipped with a snare capture element such as a snare loop **16,** system **10** or **10'** can be used in the retrieval of a vascular filter implanted in the vasculature of a patient, for example a vena cava filter implanted in a vena cava of a patient. It will be understood, however, that other uses of systems in accordance with the present disclosure, including those equipped with a snare loop, may be made. Generally in such uses, the sheath of the system **10** or **10'** (potentially after flushing the lumen of the sheath for example as described above when a flush port is included as in system **10),** can be advanced into the vasculature of a patient. The actuator button **20** or **20'** can be manipulated by hand by the user to advance and/or withdraw and/or rotate the control shaft **18** or **18',** which in turn may manipulate (e.g. including deploy) a medical device structure in the vasculature of the patient, so as to treat the patient. These as well as other operations or uses of the systems **10** or **10'** as described herein will be apparent to those skilled in the art and are contemplated in aspects of the present disclosure.

Medical device control systems **10** or **10'** or medical device control handles as described herein can be sterilized for use. For example they can be packaged in a medical package defining a sterile barrier within which they are positioned, and the package with the system **10** or **10'** or the medical device control handle can be terminally sterilized using a suitable technique such as exposure to ethylene oxide gas or radiation.

### LISTING OF CERTAIN EMBODIMENTS

The following provides an enumerated, non-limiting listing of some embodiments disclosed herein.
Embodiment 1. A system for hand-held control of a medical device structure, comprising:
   a control handle including a handle housing and an actuator movably mounted to the handle housing and arranged for manual operation by a hand of a user;
   a control shaft connected to the actuator;
   a sheath attached to a distal end of the handle housing and having a lumen extending to a distal opening of the sheath, with the control shaft extending from the handle housing and into the lumen of the sheath;
   a medical device structure attached to the control shaft, with the actuator being operable by the hand of the user to cause movement of the control shaft distally relative to the handle housing so as to deploy the medical device structure from the distal opening of the sheath and proximally relative to the handle housing so as to retract the medical device structure into the distal opening of the sheath;
   a seal element mounted in the handle housing, with the control shaft extending through the seal element, wherein the seal element is cooperable with the control shaft to maintain a seal against the control shaft during said movement of the control shaft; and
   a flush port opening in fluid communication with the lumen of the sheath, optionally wherein the flush port opening is on the control handle.
Embodiment 2. The system of Embodiment 1, wherein the seal element sealingly interrupts a fluid passage extending from the distal opening of the sheath, through the lumen of the sheath and into an interior space of the handle housing.
Embodiment 3. The system of Embodiment 1 or 2, wherein the seal element comprises at least a first opening therein, with said control shaft received through said first opening and being longitudinally slidable and rotatable relative to said first opening.
Embodiment 4. The system of any one of Embodiments 1 to 3, wherein the seal element comprises at least a first elastomeric disc.
Embodiment 5. The system of any one of Embodiments 1 to 4, comprising at least one of, or both of:
   a. a seal assembly mounted within the handle housing, the seal assembly including the seal element received against a seal body portion defining a first lumen, the seal assembly optionally also including a seal cap attached to the seal body portion and pressing the seal element against the seal body portion; and
   b. a flush port assembly mounted within the handle housing, the flush port assembly including a flush port body including a first flush port body portion defining a first flush lumen portion and a second flush port body portion extending transversely to the first flush port body portion and defining a second flush lumen portion extending from the flush port opening and intersecting and fluidly communicating with the first flush lumen portion, wherein the second flush port body portion provides a flush port stem.
Embodiment 6. The system of Embodiment 5, wherein:
   a. the handle housing has one or more inwardly-directed walls that contact and positionally fix the seal assembly relative to the handle housing, optionally wherein the one or more inwardly-directed walls contact and positionally fix the seal assembly against distal, proximal, lateral and rotational movement relative to the handle housing; and/or
   b. the handle housing has one or more inwardly-directed walls that contact and positionally fix the flush port assembly relative to the handle housing, optionally wherein the one or more inwardly-directed walls contact and positionally fix the flush port assembly against distal, proximal, lateral and rotational movement relative to the handle housing.
Embodiment 7. The system of Embodiment 5 comprising the seal assembly; or, the system of Embodiment 6 comprising the seal assembly, optionally wherein the seal assembly includes a mount portion having a non-circular external profile and the one or more inwardly-directed walls of the handle housing includes a mount wall that contacts surfaces of the non-circular external profile of the mount portion so as to prevent rotation of the seal assembly relative to the handle housing.
Embodiment 8. The system of Embodiment 5 comprising the flush port assembly; or, the system of Embodiment 6 comprising the flush port assembly, optionally wherein the flush port assembly includes a mount portion having a non-circular external profile and the one or more inwardly-directed walls of the handle housing includes a mount wall that contacts surfaces of the non-circular external profile of the mount portion so as to prevent rotation of the flush port assembly relative to the handle housing.
Embodiment 9. The system of Embodiment 5 or 6, comprising both the seal assembly and the flush port assembly.
Embodiment 10. The system of Embodiment 9, wherein the flush port assembly and the seal assembly are subcomponents of a flush port/seal assembly.
Embodiment 11. The system of Embodiment 10, wherein the seal body portion and the flush port body are segments of an integral flush port/seal body.
Embodiment 12. The system of Embodiment 10 or 11, wherein the handle housing has one or more inwardly-directed walls that contact and positionally fix the flush port/seal assembly relative to the handle housing, optionally wherein the one or more inwardly-directed walls contact and positionally fix the flush port/seal assembly against distal, proximal, lateral and rotational movement relative to the handle housing.
Embodiment 13. The system of Embodiment 12, wherein the handle housing is composed of at least a first handle housing portion and a second handle housing portion connected together.
Embodiment 14. The system of Embodiment 13, wherein the first handle housing portion includes a first inwardly-directed wall portion and the second handle housing portion includes a second inwardly-directed wall portion, wherein the first and second inwardly-directed wall portions align with one another and have respective inward end surfaces that contact and cradle an exterior surface of the flush port/seal assembly.
Embodiment 15. The system of Embodiment 14, wherein said respective inward end surfaces form a collar that surrounds said exterior surface.
Embodiment 16. The system of any one of Embodiments 5 to 15, wherein the flush port stem extends at least partly exterior of the handle housing.
Embodiment 17. The system of any one of Embodiments 5 to 16, wherein the flush port stem is positioned proximate to a distal end of the handle housing.
Embodiment 18. The system of any one of Embodiments 10 to 15, wherein the flush port/seal assembly includes a mount portion having a non-circular external profile, and wherein the handle housing includes one or more internal walls that contact surfaces of the non-circular external profile so as to prevent rotation of the flush port/seal assembly relative to the handle housing, optionally wherein the non-circular external profile is a polygonal profile.
Embodiment 19. The system of Embodiment 18, wherein the seal body portion and the flush port body are segments of an integral flush port/seal body, and wherein the mount portion is defined by the integral flush port/seal body.
Embodiment 20. The system of any one of Embodiments 5 to 19, wherein the flush port assembly includes a connector cap attached to the flush port body and securing a distal end of the sheath to the flush port body.
Embodiment 21. The system of Embodiment 20, wherein the connector cap extends at least partly exterior and distal of a distal end of the handle housing.
Embodiment 22. The system of any preceding Embodiment, wherein the actuator is also rotatable by the hand of the user to cause rotation of the control shaft relative to the handle housing so as to rotate the medical device structure.
Embodiment 23. The system of Embodiment 22, wherein the actuator is a thumb wheel.
Embodiment 24. The system of any preceding Embodiment, also comprising a locking mechanism for locking the actuator against movement relative to the handle housing.
Embodiment 25. The system of Embodiment 24, wherein the locking mechanism includes a lock member pivotally connected to the handle housing and pivotable to a first position that contacts and prevents movement of the actuator relative to the handle housing and to a second position that allows movement of the actuator relative to the handle housing.
Embodiment 26. The system of any preceding Embodiment, wherein the medical device structure is a snare element, preferably a vascular snare element.
Embodiment 27. The system of Embodiment 26, wherein the snare element includes a snare loop.
Embodiment 28. The system of Embodiment 27, wherein the snare loop is configured to extend transversely to a longitudinal axis of the control shaft when the snare loop is deployed out of the distal opening of the sheath.
Embodiment 29. The system of any preceding Embodiment, wherein the actuator is rotatable relative to the handle housing and slidable longitudinally relative to the handle housing independent of rotation of the actuator.
Embodiment 30. The system of Embodiment any preceding Embodiment, wherein the flush port opening is in fluid communication with the seal element.
Embodiment 31. The system of any preceding Embodiment, wherein the handle housing includes a finger rest projecting from a side of the handle housing.
Embodiment 32. The system of any preceding Embodiment, wherein the handle housing defines an elongate longitudinally-extending opening along which the actuator moves, optionally wherein a first portion of the actuator is positioned within the handle housing and a second portion of the actuator extends out of the elongate longitudinally-extending opening.
Embodiment 33. The system of any preceding Embodiment, wherein the control shaft includes a control shaft portion suspended in the handle housing and occurring between the actuator and the seal element.
Embodiment 34. The system of any preceding Embodiment, further comprising a flush port cap for sealing the flush port opening.
Embodiment 35. A method for preparing a system prior to use on a patient, comprising:
   providing a system according to any one of Embodiments 1 to 34; and
   passing a flush liquid into the flush port opening, through the lumen of the sheath, and out the distal opening of the sheath.
Embodiment 36. A method for treating a patient using a medical device, comprising:
   providing a system according to any one of Embodiments 1 to 34;
   passing a flush liquid into the flush port, through the lumen of the sheath, and out the distal opening of the sheath;
   after said passing a flush liquid, introducing the sheath into a vascular vessel of the patient;
   operating the actuator by hand to cause movement of the control shaft distally relative to the handle housing so as to deploy the medical device structure from the distal opening of the sheath; and
   treating the patient with the medical device structure.
Embodiment 37. The method of Embodiment 36, wherein the medical device structure is a vascular snare element, and wherein the treating comprises securing and removing an implant from the vascular vessel with the vascular snare element.
Embodiment 38. The method of Embodiment 37, wherein the implant is a vascular filter.
Embodiment 39. The method of Embodiment 38, wherein the vascular vessel is an inferior vena cava of the patient, and wherein the vascular filter is an inferior vena cava filter.
Embodiment 40. A medical device control handle for hand-held control of a medical device structure that is connected to a control shaft and deployable from a lumen of a sheath, the medical device control handle comprising:
   a handle housing;
   an actuator movable relative to the handle housing and arranged for manual operation by a hand of a user, the actuator being connectable to the control shaft; and
   at least one of, or both of:
      a. a seal assembly mounted in the handle housing and configured to maintain a seal against an outer surface of a control shaft for a medical device, the seal assembly including a seal body defining a lumen therein and a seal element received against the seal body; and
      b. a flush port assembly mounted in the handle housing, the flush port assembly including a flush port body having a first flush port body portion defining a first flush lumen portion therein configured to fluidly communicate with a sheath lumen and a second flush port body portion extending transverse to the first flush port body portion and providing a flush port stem defining a second flush lumen portion therein and
   extending to a flush port opening, the second flush lumen portion intersecting and fluidly communicating with the first flush lumen portion.
Embodiment 41. The medical device control handle of Embodiment 40, including the seal assembly, the seal assembly optionally also including a seal cap attached to the seal body so as to press the seal element against the seal body portion.
Embodiment 42. The medical device control handle of Embodiment 40, including the flush port assembly.
Embodiment 43. The medical device control handle of Embodiment 40, including both the seal assembly and the flush port assembly.
Embodiment 44. The medical device control handle of any one of Embodiments 40 to 43, wherein the handle housing has one or more inwardly-directed walls that contact and positionally fix the flush port assembly when present or the seal assembly when present relative to the handle housing.
Embodiment 45. The medical device control handle of any one of Embodiments 40 to 44, including both the seal assembly and the flush port assembly, and wherein the flush port assembly and the seal assembly are subcomponents of a flush port/seal assembly.
Embodiment 46. The medical device control handle of Embodiment 45, wherein the seal body and the flush port body are segments of an integral flush port/seal body.
Embodiment 47. The medical device control handle of Embodiment 45 or 46, wherein the handle housing has one or more inwardly-directed walls that contact and positionally fix the flush port/seal assembly relative to the handle housing, optionally wherein the one or more inwardly-directed walls contact and positionally fix the flush port/seal assembly against distal, proximal, lateral and rotational movement relative to the handle housing.
Embodiment 48. The medical device control handle of Embodiment 47, wherein the handle housing is composed of at least a first handle housing portion and a second handle housing portion connected together.
Embodiment 49. The medical device control handle of Embodiment 48, wherein the first handle housing portion includes a first inwardly-directed wall portion and the second handle housing portion includes a second inwardly-directed wall portion, wherein the first and second inwardly-directed wall portions align with one another and have respective inward end surfaces that contact and cradle an exterior surface of the flush port/seal assembly.
Embodiment 50. The medical device control handle of Embodiment 49, wherein the seal body portion and the flush port body are segments of an integral flush port/seal body and wherein said exterior surface is on the integral flush port/seal body.
Embodiment 51. The medical device control handle of Embodiment 49 or 50, wherein said inward end surfaces form a collar that surrounds said exterior surface.
Embodiment 52. The medical device control handle of Embodiment 48, wherein the flush port/seal assembly includes a mount portion having a non-circular external profile, and wherein the handle housing includes one or more internal walls that contact surfaces of the non-circular external profile so as to prevent rotation of the flush port/seal assembly relative to the handle housing, optionally wherein the non-circular external profile is a polygonal profile.
Embodiment 53. The medical device control handle of any one of Embodiments 40 to 52, wherein the flush port assembly includes a connector cap attached to the first flush port body portion and configured to secure a distal end of the sheath to the first flush port body portion.
Embodiment 54. The medical device control handle of Embodiment 53, wherein the connector cap extends at least partly exterior and distal of a distal end of the handle housing.
Embodiment 55. The medical device control handle of any one of Embodiments 40 to 54, wherein the actuator is movable longitudinally relative to the handle housing.
Embodiment 56. The medical device control handle of any one of Embodiments 40 to 55, wherein the actuator is rotatable by the hand of the user.
Embodiment 57. The medical device control handle of Embodiment 56, wherein the actuator is a thumb wheel.
Embodiment 58. The medical device control handle of any one of Embodiments 40 to 57, also comprising a locking mechanism for locking the actuator against movement relative to the handle housing.
Embodiment 59. A medical device control system including a medical device control handle according to any one of Embodiments 40 to 58, the sheath, the control shaft, and the medical device structure attached to the control shaft.
Embodiment 60. The system of Embodiment 59, which is also a system according to any one of Embodiments 1 to 34.

The uses of the terms "a" and "an" and "the" and similar references herein (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein, is intended merely to better illuminate embodiments of the disclosure and does not pose a limitation on the scope of the disclosure unless otherwise claimed. No language in the specification should be construed as indicating any non-claimed element as essential to the practice of the products or methods defined by the claims.

While embodiments of the disclosure have been illustrated and described in detail in the drawings and foregoing description, the same is to be considered as illustrative and not restrictive in character, it being understood that only some embodiments have been shown and described and that all changes and modifications that come within the spirit of the disclosures herein are desired to be protected.

## Claims

1. A system for hand-held control of a medical device structure, comprising:
a control handle including a handle housing and an actuator movably mounted to the handle housing and arranged for manual operation by a hand of a user;
a control shaft connected to the actuator;
a sheath attached to a distal end of the handle housing and having a lumen extending to a distal opening of the sheath, with the control shaft extending from the handle housing and into the lumen of the sheath;
a medical device structure attached to the control shaft, with the actuator being operable by the hand of the user to cause movement of the control shaft distally relative to the handle housing so as to deploy the medical device structure from the distal opening of the sheath and proximally relative to the handle housing so as to retract the medical device structure into the distal opening of the sheath;
a seal element mounted in the handle housing, with the control shaft extending through the seal element, wherein the seal element is cooperable with the control shaft to maintain a seal against the control shaft during said movement of the control shaft, optionally wherein the seal element sealingly interrupts a fluid passage extending from the distal opening of the sheath, through the lumen of the sheath and into an interior space of the handle housing; and
a flush port opening in fluid communication with the lumen of the sheath, optionally wherein the flush port opening is on the control handle.

2. The system of claim 1, wherein the seal element comprises at least a first opening therein, with said control shaft received through said first opening and being longitudinally slidable and rotatable relative to said first opening, optionally wherein the seal element comprises at least a first elastomeric disc.

3. The system of claim 1 or 2, comprising at least one of:
a. a seal assembly mounted within the handle housing, the seal assembly including the seal element received against a seal body portion defining a first lumen, the seal assembly optionally also including a seal cap attached to the seal body portion and pressing the seal element against the seal body portion; and
b. a flush port assembly mounted within the handle housing, the flush port assembly including a flush port body including a first flush port body portion defining a first flush lumen portion and a second flush port body portion extending transversely to the first flush port body portion and defining a second flush lumen portion extending from the flush port opening and intersecting and fluidly communicating with the first flush lumen portion, wherein the second flush port body portion provides a flush port stem, for example wherein the system comprises both the seal assembly and the flush port assembly, optionally wherein the flush port assembly and the seal assembly are subcomponents of a flush port/seal assembly.

4. The system of claim 3, wherein:
a. the handle housing has one or more inwardly-directed walls that contact and positionally fix the seal assembly relative to the handle housing, optionally wherein the one or more inwardly-directed walls contact and positionally fix the seal assembly against distal, proximal, lateral and rotational movement relative to the handle housing; and/or
b. the handle housing has one or more inwardly-directed walls that contact and positionally fix the flush port assembly relative to the handle housing, optionally wherein the one or more inwardly-directed walls contact and positionally fix the flush port assembly against distal, proximal, lateral and rotational movement relative to the handle housing.

5. The system of claim 3 comprising the seal assembly; or, the system of claim 4 comprising the seal assembly, optionally wherein the seal assembly includes a mount portion having a non-circular external profile and the one or more inwardly-directed walls of the handle housing includes a mount wall that contacts surfaces of the non-circular external profile of the mount portion so as to prevent rotation of the seal assembly relative to the handle housing.

6. The system of claim 3 comprising the flush port assembly; or, the system of claim 4 comprising the flush port assembly, optionally wherein the flush port assembly includes a mount portion having a non-circular external profile and the one or more inwardly-directed walls of the handle housing includes a mount wall that contacts surfaces of the non-circular external profile of the mount portion so as to prevent rotation of the flush port assembly relative to the handle housing.

7. The system of any preceding claim, also comprising a locking mechanism for locking the actuator against movement relative to the handle housing.

8. The system of any preceding claim, wherein the medical device structure is a snare element that includes a snare loop configured to extend transversely to a longitudinal axis of the control shaft when the snare loop is deployed out of the distal opening of the sheath, preferably a vascular snare element.

9. The system of any preceding claim, wherein the actuator is rotatable relative to the handle housing and slidable longitudinally relative to the handle housing independent of rotation of the actuator.

10. A method for preparing a system prior to use on a patient, comprising:
providing a system according to any one of claims 1 to 9; and
passing a flush liquid into the flush port opening, through the lumen of the sheath, and out the distal opening of the sheath.

11. A medical device control handle for hand-held control of a medical device structure that is connected to a control shaft and deployable from a lumen of a sheath, the medical device control handle comprising:
a handle housing;
an actuator movable relative to the handle housing and arranged for manual operation by a hand of a user, the actuator being connectable to the control shaft; and
at least one of, or both of:
c. a seal assembly mounted in the handle housing and configured to maintain a seal against an outer surface of a control shaft for a medical device, the seal assembly including a seal body defining a lumen therein and a seal element received against the seal body; and
d. a flush port assembly mounted in the handle housing, the flush port assembly including a flush port body having a first flush port body portion defining a first flush lumen portion therein configured to fluidly communicate with a sheath lumen and a second flush port body portion extending transverse to the first flush port body portion and providing a flush port stem defining a second flush lumen portion therein and extending to a flush port opening, the second flush lumen portion intersecting and fluidly communicating with the first flush lumen portion.

12. The medical device control handle of claim 11, including at least one of:
a. the seal assembly, the seal assembly optionally also including a seal cap attached to the seal body so as to press the seal element against the seal body portion; and
b. the flush port assembly,
for example wherein the medical device control handle includes both the seal assembly and the flush port assembly, and the flush port assembly and the seal assembly are subcomponents of a flush port/seal assembly, optionally wherein the seal body and the flush body are segments of an integral flush port/seal body.

13. The medical device control handle of claims 11 or 12, wherein the handle housing has one or more inwardly-directed walls that contact and positionally fix the flush port assembly when present or the seal assembly when present relative to the handle housing.

14. The medical device control handle of any one of claims 11 to 13, wherein the actuator is movable longitudinally relative to the handle housing and rotatable by the hand of the user, and/or wherein the system also comprises a locking mechanism for locking the actuator against movement relative to the handle housing.

15. A medical device control system including a medical device control handle according to any one of claims 11 to 14, the sheath, the control shaft, and the medical device structure attached to the control shaft.
